(19) Europäisches Patentamt
European Patent Office
Office européen des brevets

(11) Numéro de publication : 0 526 301 A1

# (12) DEMANDE DE BREVET EUROPEEN

(21) Numéro de dépôt : 92402075.3

(22) Date de dépôt : 17.07.92

(51) Int. Cl.[5] : C07C 69/675, C07C 67/03, C11D 1/04, C11D 1/66, A61K 7/075, C07C 67/30

(30) Priorité : 24.07.91 FR 9109342

(43) Date de publication de la demande : 03.02.93 Bulletin 93/05

(84) Etats contractants désignés : AT BE CH DE DK ES FR GB GR IT LI LU MC NL PT SE

(71) Demandeur : AGRO INDUSTRIE RECHERCHES ET DEVELOPPEMENTS (A.R.D.)
27/29 rue Châteaubriand
F-75008 Paris (FR)

(71) Demandeur : ZSCHIMMER & SCHWARZ GmbH & Co. CHEMISCHE FABRIKEN
Max-Schwarz-Strasse 4-5 Postfach 2179
W-5420 Lahnstein (DE)

(72) Inventeur : Petit, Serge
120, rue Maréchal Joffre
F-60150 Montmacq (FR)
Inventeur : Ralainirina, Robert
11, rue René Fonck
F-80080 Amiens (FR)
Inventeur : Favre, Serge
1,rue du Général Debeney, Résidence Le Plessis
F-60200 Compiegne (FR)
Inventeur : Baynast, Régis de
35, rue de l'Ermitage
F-78000 Versailles (FR)

(74) Mandataire : Bourgognon, Jean-Marie et al
Cabinet Flechner 22, Avenue de Friedland
F-75008 Paris (FR)

## (54) Alcoyl galactarates, leurs procédés de préparation et leurs applications.

(57) Les alcoyl galactarates de formule :

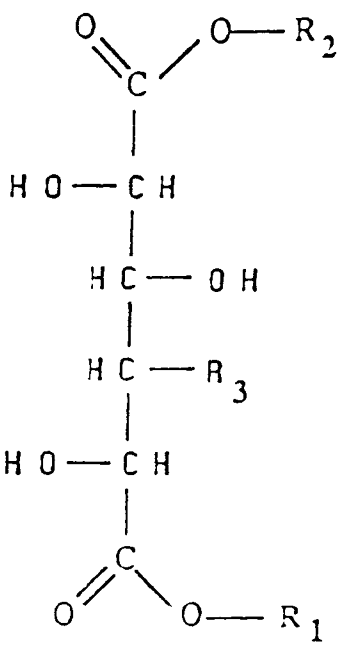

(I)

dans laquelle R1 est alcoyle ou alcényle ayant de 1 à 22 atomes de carbone et, ou bien R2 est, quand il

EP 0 526 301 A1

est différent de R1, l'hydrogène, alcoyle ou alcényle ayant de 1 à 22 atomes de carbone, et quand il est identique à R2, alcoyle ou alcényle ayant de 7 à 22 atomes de carbone, et R3 est hydroxy, ou bien R3 forme avec R2 une liaison de valence entre les atomes qui les pertent, et leurs sels avec des métaux alcalins, alcalino-teneux ou d'ammonium sont des agents tensio-actifs.

La présente invention concerne des alcoyl galactarates, leurs procédés de préparation et leurs diverses applications, notamment comme agents tensio-actifs non ioniques ou anioniques.

Les technologies d'hydrolyse enzymatique et de fractionnement de la matière première végétale (betteraves, chicorées...) mises au point par l'une des demanderesse (WO 8800243 A1, 14 janvier 1988 - CA 110 (9) 73855 g) permettent l'accès à un acide D-galacturonique bon marché et en abondance.

Son oxydation selon les techniques classique (F. Erlich, Chem. Ber., 1929, 62 197 et M.S. Isbell, US 2.238.114 4 janv. 1944) conduit au diacide correspondant, l'acide galactarique ou mucique, qui est un substrat de choix pour l'obtention d'agents tensio-actifs non-ioniques ou anioniques.

L'invention vise les alcoyl galactarates de formule:

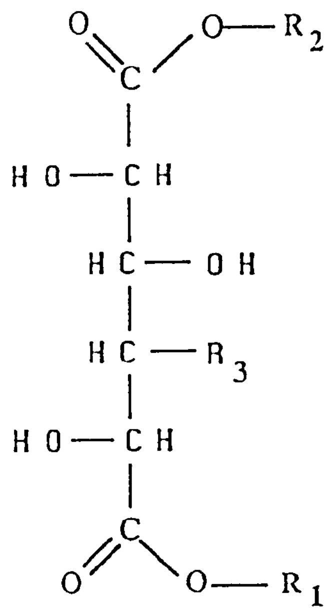

(I)

dans laquelle R1 est alcoyle ou alcényle ayant de 1 à 22 atomes de carbone et, ou bien, R2 est, quand il est différent de R1, l'hydrogène, alcoyle ou alcényle ayant de 1 à 22 atomes de carbone, et, quand il est identique à R2, alcoyle ou alcényle ayant de 7 à 22 atomes de carbone, et R3 est hydroxy, ou bien R3 forme avec R2 une liaison de valence entre les atomes qui les portent.

L'invention vise également, quand R2 est l'hydrogène, les sels des acides correspondants; ces sels pouvant être des sels de métaux alcalins, par exemple le sodium, le potassium, des sels de métaux alcalino-terreux, par exemple le magnésium, ou des sels d'ammonium quaternaires, par exemple l'ion ammonium, l'ion méthyl-2-hydroxyméthyl-2 éthyl ammonium, l'ion tri(hydroxyméthyl)méthyl ammonium, l'ion di(hydroxyméthyl)2,2 éthyl ammonium et les ammoniums de formule générale R4 R5 R6 R7 $N^+$ où R4, R5, R6, R7 est soit l'hydrogène, mais pas tous simultanément, soit un bas alcoyle.

Les alcoyl galactarates de formule (I) où R1 = R2 sont nouveaux quand R1, R2, ont une condensation en carbone supérieure à 6; on mentionne à titre de simple conjecture, le dioctyl galactarate (Alex Jahn, Ger 878, 863, June 8, 1953) sans en donner le procédé de préparation. On a également conjoncturé par sa formule le composé de formule (I) où R1 = éthyle et R3 forme avec R2 une liaison de valence entre les atomes qui les portent (Carbohydrate Research, 98, 1981, 203-208, Peter D-Hoagland). Cette 6-éthyle galactarate-1,4 lactone est présentée comme étant préparée par des procédés connus par l'article de B.A. Lewis, F. Smith et A.M. Stephen ("Method in carbohydrate chemistry" R.L.-Whistler et M.L. Wolfrom, Eds. vol. II, Academic Press, New-York, 1963, 38-46), mais quand on se reporte à cet article, seule le 2,3,5-tri-O-méthyl-galactarate 1,4-lactone est décrite, c'est-à-dire un composé dont les hydroxydes sont protégés par formations d'éthers méthyliques dont on sait qu'ils sont difficiles à cliver sans saponifier la fonction ester. De plus, cette monolactone est présentée comme un produit transitoire, obtenu in situ et non séparé, dans la préparation de diamides galactariques.

On préférera, parmi les alcoyl galactarates décrits selon l'invention, ceux de formule (I) dans lesquels R1 est alcoyle ou alcényle ayant de 8 à 18 atomes de carbone et, ou bien R2 est, quand il est différent de R1,

l'hydrogène, alcoyle ou alcényle ayant de 1 à 22 atomes de carbone, et, quand il est identique à R2, alcoyle ou alcényle ayant de 9 à 22 atomes de carbone, et R3 est hydroxy, ou bien R3 forme avec R2 une liaison de valence entre les atomes qui les portent.

L'invention vise également un procédé de préparation d'alcoyl galactarates de formule (I) caractérisé en ce qu'il consiste :

- Pour préparer les alcoyl galactarates de formule (I) dans lesquels R1 et R2 sont identiques, à transestérifier un galactarate de dialcoyle symétrique ayant une condensation en carbone de chacune des ses chaînes alcoyles égale à n, n entier inférieure à 7 et de préférence compris entre 2 et 4.

Ce galactarate de dialcoyle symétrique peut être obtenu par estérification de l'acide galactarique par un excès d'un alcool de formule CnH2n+1 OH, selon les protocoles classiques (B.A. Lewis, F. Smith et A.M. Stephen, "Method in carbohydrate chemistry", R.L. Whistler et M.L. Wolfrom, Eds., Vol. II, Academic Press, New-York, 1963, 38-46); le procédé consiste par exemple à opérer en présence d'acide sulfurique et au reflux de l'éthanol absolu pendant 24 heures pour la préparation du galactarate de diéthyle.

Cette transestérification, par plus d'un équivalent molaire, d'un alcool de formule R1OH, R1 ayant une condensation en carbone supérieure à n est conduite en présence d'un catalyseur acide ou basique.

- Pour préparer les alcoyl galactarates de formule (I) dans lesquels R1 n'est pas l'hydrogène et est différent de R2, à transestérifier un galactarate de dialcoyle symétrique ayant une condensation en carbone de chacune de ses chaînes égale à n, n entier inférieur à 7, et de préférence compris entre 2 et 4 où bien par un équivalent molaire d'un alcool de formule $R_2OH$, $R_2$ ayant une condensation en carbone supérieure à n, en présence d'un catalyseur acide; ou bien par un mélange d'au moins deux alcools de formules respectives $R_1OH$ et $R_2OH$, tant R1 que R2 ayant une condensation en carbone supérieure à n, et la somme des moles des alcools de formule $R_1OH$ et $R_2OH$ engagés dans la réaction étant supérieure au nombre de moles de dialcoyl galactarate symétrique mis en réaction et de préférence égal au double, ceci en présence d'un catalyseur acide.

La réaction est conduite en distillant partiellement l'alcool formé pendant un temps compris entre 1 heure et 10 heures, et de préférence 2 à 5 heures, puis on prolonge le chauffage, sans distillation jusqu'à l'obtention du galactarate attendu.

- Pour préparer les alcoyl galactarates cyclisés de formule I dans lesquels R3 forme avec R2 une liaison de valence,

. ou bien à transestérifier un galactarate de dialcoyle symétrique ayant une condensation en carbone de chacune de ses chaînes égale à n,n entier inférieur à 7, et de préférence compris entre 2 et 4, par au plus un équivalent molaire d'un alcool R1OH, R1 ayant une condensation en carbone supérieure à n, en présence d'un catalyseur acide ou basique, puis à éliminer totalement par distillation, l'alcool de formule $C_nH_{2n} + {}_1OH$ résultant de la transestérification et la cyclisation,

. ou bien, à saponifier unilatéralement puis à cycliser, en milieu basique, un galactarate de dialcoyle symétrique ayant une condensation en carbone de chacune de ses chaînes égales à m, m entier compris entre 2 et 22 et obtenu selon l'invention,

. ou bien, à estérifier en milieu acide ou basique, par un équivalent molaire d'un alcool de formule R1OH, R1 ayant une condensation en carbone allant de 1 à 22 atomes, la monolactone de l'acide galactarique obtenu, soit selon les protocoles classiques de la littérature (B.A. Lewis, F. Smith et A.M. Stephen, "Method in carbohydrates chemistry" R.L. Whistler et M.L. Wolfrom, Eds. Vol. II Academic Press. New-York, 1963, 38-46) c'est-à-dire par chauffage au reflux de l'eau pendant 30 minutes, concentration rapide puis extraction à l'acétone (rendement 77%), soit par chauffage, de 1 à 12 H et de préférence de 3 à 6 heures, dans du diméthylformamide à 130°C (rendement 100%).

. ou bien à chauffer un dialcoyle galactarate symétrique de formule I dans un solvant organique et en présence d'un catalyseur acide ou basique.

- Pour préparer les alcoyl galactarate de formule (I) dans lesquels R est l'hydrogène, soit à mettre en présence d'eau un alcoyle galactarate cyclisé dans lequel R3 forme avec R2 une liaison de valence, soit à traiter ce dernier par un équivalent d'une base en solution aqueuse pour obtenir la forme de sel de l'acide correspondant à l'ouverture de la fonction lactone.

En pratique, le procédé consiste également à:

- à éventuellement ajouter au milieu réactionnel, de 2 à 20 équivalents en poids par rapport au substrat d'un solvant pouvant être de type étheroxyde, tel le tetrahydrofuranne, le dioxanne, les éthers dialcoyles de l'éthylène glycol, par exemple l'éther diméthylique de l'éthylène glycol, l'éther diméthylique du diéthylène glycol, un hydrocarbure halogéné tel le dichlorométhane, le chloroforme, le dichloroéthane, un ester tel que l'acétate diéthyle, l'acétate de propyle ou l'acétate de bytyle, un solvant nitré tel que le nitrométhane, le nitroéthane, le nitro-2-propane, un solvant de la famille des amides tels que le N-méthyl formamide, le N,N-diméthylformamide, le N,N diméthyl acétamide, la N-méthyl -2-pyrrolidone, un nitrile tel que l'acétonitrile, ou un alcane, de préférence l'hexane, l'heptane ou l'octane, ou un solvant organique tel que le toluène ou le xylène ; on peut utiliser également un mélange de deux ou plusieurs de ces solvants, mais on préfèrera conduire la réaction en l'absence de solvant.

On préfèrera un solvant de type dialcoyle oxy alcoyle, notamment l'éther diméthylique de l'éthylène glycol.

- à préférer, lorsque l'on doit faire réagir plus d'un équivalent molaire d'un alcool ou d'un mélange d'alcool, l'utilisation de deux équivalents molaires de cet alcool ou du mélange de ces alcools par rapport au substrat,
- à utiliser par rapport au substrat de 0,01 à 2 équivalents molaires,

soit d'un catalyseur acide, tel que l'acide chlorhydrique, l'acide sulfurique, l'acide méthyl sulfonique, l'acide benzène sulfonique, l'acide paratoluène sulfonique, l'acide camphre sulfonique, salicylsulfonique, succinyl sulfonique, les acides perhalohydriques tels que l'acide perchlorique des métaux tels que le cuivre ou le fer, leurs oxydes ou leurs sels, comme leurs halogénures, des halogènes comme l'iode, des pentahalogénures d'antimoine, notamment des pentachlorures ou pentafluorures, ou des sulfates de titane.

Cette catalyse peut être effectuée par 0,05 à 6 équivalents pondéraux d'une résine sulfonique sous sa forme H+ ou d'une argile acide. On préfère l'acide sulfurique, l'acide méthylsulfonique, succinyl sulfonique ou salicylsulfonique.

soit d'un catalyseur basique tel un carbonate, un hydrogénocarbonate, un hydroxyde, soit de métal alcalin, de préférence le sodium ou le potassium, soit d'un alcalino-terreux, de préférence magnésium, soit d'un ammonium tel que l'hydroxyde de méthyl-2 hydroxyméthyl-2 éthyl ammonium, l'hydroxyde de tri (hydroxyméthyl) méthyl ammonium, l'hydroxyde de di (hydroxyméthyl)-2,2 éthyl ammonium, l'ammoniaque, les hydroxydes d'ammonium quaternaires de formule $R_4R_5R_5R_7N^+$ où $R_4R_5R_6R_7$ est soit l'hydrogène, mais pas simultanément,

soit un bas alkyl ou un catalyseur basique tel qu'une résine anionique, notamment de type ammonium et sous sa forme hydroxyde ;

- à effectuer les réactions à des températures comprises entre 25 et 150°C et de préférence entre 70 et 130°C, pendant une durée de 1 heure à 3 jours et, de préférence, de + 1 heure à 30 heures;
- à effectuer les réactions au reflux du solvant qui peut être l'alcool en excès, ou du mélange des solvants décrits selon l'invention;
- à effectuer les réactions sans distillation et/ou avec distillation de l'alcool volatil formé au cours des réactions de transestérification et/ou de cyclisation ;
- à filtrer le catalyseur acide ou basique lors d'une catalyse hétérogène, ou à neutraliser le catalyseur acide, puis à filtrer son sel lors d'une catalyse homogène. La neutralisation est faire par exemple par un hydrogénocarbonate de métal alcalin, notamment l'hydrogénocarbonate de sodium ou de potassium,
- à évaporer le solvant et/ou l'excès d'alcool pour récupérer les alkyls galactarates de formule (I).

Le procédé est également caractérisé par le fait qu'il donne d'excellents rendements en alcoyl galactarates de formule (I), généralement quantitatifs ; ceci notamment pour les alkyls galactarates symétriques de formule (I) déjà décrits dans la littérature (Method in carbohydrate chemistry, R. L. Whistler et H.L. - Wolfrom, Eds. vol II, Academic Press, New-York 1963, pp 38-46 ; Günter Drefahl et Bertlod Gross ; J. Prakt Chem. 4, 1, 153-156, 1955 ; F. Carlson, J. Am. Chem. soc., 1948, p 2609) et préparés par les auteurs par addition d'un excès de l'alcool correspondant sur l'acide galactarique en présence d'un catalyseur d'acide tel que l'acide sulfurique, l'acide chlorhydrique, l'acide paratoluène sulfonique, en une résine cationique du type Wafatit X, et en présence ou non de toluène ou de benzène. Les rendements selon cette approche directe, comme le confirment les exemples comparatifs ci-après (exemples comparatifs de 1 à 9) diminuent avec l'augmentation de la longueur de la chaîne carbonée de l'alcool mis en réaction. Ils montrent que la réaction devient difficile, voire impossible lorsque l'on utilise des alcools de condensation en carbone supérieure à 8.

L'invention fait appel comme substrat de base non pas à l'acide galactarique lui-même, mais à un galactarate de dialcoyle symétrique ayant une condensation en carbone de chacune de ses chaînes alcolyes égale à n, n entier inférieur à 7 et de préférence compris entre 2 et 4, ou le monolactone de l'acide galactarique. Ces substrats, plus lipophiles réagissent plus vite et avec de meilleurs rendements, notamment avec des alcools de haut poids moléculaire.

Elle permet de préparer avec d'excellents rendements (généralement quantitatifs) d'une part les composés déjà décrits dans la littérature et d'autre part, de synthétiser de nouveaux galactarates inaccessibles ou très difficilement accessibles avec l'approche classique directe.

Les alcoyl galactarates, suivant l'invention, peuvent être utilisés comme agents tensio-actifs. L'invention vise donc également l'utilisation d'alcoyl galactarates de formule (I)

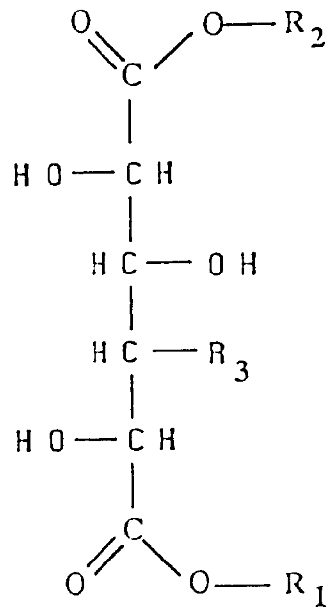

(I)

R1 étant alcoyle ou alcenyl ayant de 1 à 22 atomes de carbones et ou bien R2 est, quand il est différent de R1, l'hydrogène, alcoyle ou alcényle ayant de 1 à 22 atomes de carbone et R3 est hydroxy ou bien R3 forme avec R2 une liaison de valence entre les atomes qui les portent. L'invention vise également, quand R2 est l'hydrogène, l'utilisation des sels des acides alcoyl galactariques correspondants comme agents tensio-actifs. Ces alcoyl galactarates possèdent en particulier des propriétés de solubilisation émulsionnantes, moussantes, mouillantes ou dispersantes.

Ils peuvent être utilisés comme détergents, notamment dans des compositions lessivielles et dans des compositions de lavage de la vaisselle, des sols et des vitres, comme adoucissants et comme adjuvants de détergence dans ces mêmes compositions, aussi bien en poudre que liquides. Ils peuvent également entrer dans les compositions capillaires ou de shampooing et être utiles en cosmétologie, sous forme de pommades, crèmes et laits de beauté, afin d'adoucir et de raffermir la peau en tant que tensio-actifs doux n'agressant ni la peau ni les muqueuses. Leur pouvoir moussant et adoucissant peut être mis à profit également dans les compositions de bains moussants. Ils peuvent également entrer dans les formulations de produits alimentaires, tels que comme additifs d'huiles et de graisses, dans des crèmes mayonnaises et des sauces. Dans l'industrie, ils peuvent servir d'agents pour effectuer des polymérisations en émulsion. Ce sont également des cristaux liquides qui peuvent être utilisés en tant que tels en électro-optique et en cosmétologie sous forme de vésicules permettant de stabiliser et de vectoriser des principes actifs.

Les exemples suivants illustrent l'invention:

Exemples comparatifs de préparation de dialcoyles galactarates de formule I avec R1 ≠ R2 selon les voies directes citées dans la littérature (tableau I ci-après).

Exemples comparatifs n° 1 à 4 : estérification directe en excès d'alcool et en présence de toluène.

On porte à 110°C, pendant 24 H une suspension de 20 g d'acide galactarique dans un excès d'alcool contenant de l'acide sulfurique concentré. Après neutralisation, à l'hydrogénocarbonate de sodium, filtration et distillation de l'alcool en excès, on récupère les dialcoyles galactarates attendus.

Exemples comparatifs n° 5 à 9 : estérification directe en excès d'alcool et en présence de toluène.

On porte à 85°C pendant 40 heures une suspension de 10 g d'acide galactarique dans un excès d'alcool et en présence de 200 g de toluène et de 0,4 g d'acide paratoluène sulfonique. Après neutralisation du catalyseur à l'hydrogénocarbonate de sodium, filtration des sels formés et concentration du milieu réactionnel, on récupère les dialcoyles galactarates attendus.

| Exemple n° | Méthode | Galactarates dialcoyl formés | Quantité d'alcool ajoutée (ml) | Rendements (%) |
|---|---|---|---|---|
| 1 | Estérification | dibutyl | 200 | 82 |
| 2 | directe | dihexyl | 1600 | 70 |
| 3 | sans | dioctyl | 3000 | 55 |
| 4 | solvant | didécyl | 5000 | 20 |
| 5 | Estérification | dihexyl | 60 | 73 |
| 6 | directe | dioctyl | 80 | 60 |
| 7 | en | didécyl | 100 | 35 |
| 8 | présence | didodécyl | 120 | 25 |
| 9 | toluène | dihexadécyl | 140 | 20 |

TABLEAU I : Préparation d'alcoyl galactarates de formule I avec R1 = R2, selon la voie directe citée dans la littérature.

Exemple n° 10 : préparation, selon l'invention, du galactarate de didodécyle (composé de formule I tel que R1 = R2 = dodécyl et R3 = hydroxy).

On chauffe à 85°C pendant 24 heures, un mélange contenant 10 g de galactarate de diéthyle, 16,9 ml de n. dodécanol, 1 ml d'acide sulfurique concentré et 100 ml de diméthoxyéthane (DME). Après distillation partielle de l'éthanol formé (85°C pendant 1 H), refroidissement et neutralisation du milieu réactionnel à l'hydrogénocarbonate de sodium, on filtre le sel formé et on concentre sous vide pour obtenir, avec 99,8 % de rendement, le didodécyl galactarate attendu sous forme d'un précipité blanc.

RMN : $\partial^{13}C$ dans pyridine d5

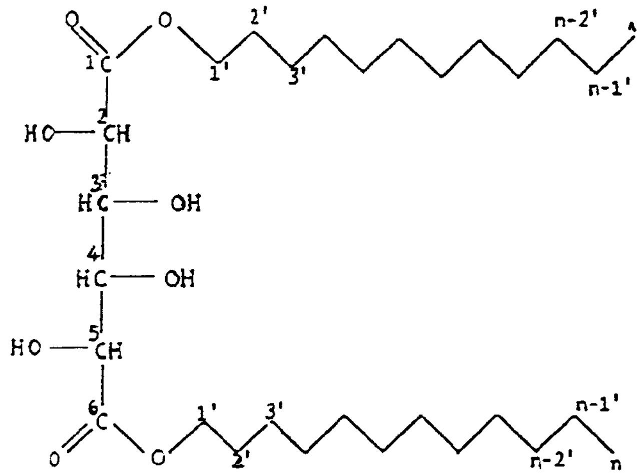

| Cn | ∂ ppm |
|---|---|
| 1,6 | 175,18 |
| 2,5 | 73,05 |
| 3,4 | 72,14 |
| 1' | 64,99 |
| 2' | 49,63 |
| 3' | 34,09 |
| n-2' | 26,05 |
| n-1' | 22,90 |
| 2n | 14,23 |

Exemples n° 11 à 14 :

Selon un protocole analogue à celui décrit dans l'exemple n° 10, sont préparés les dialcoyles galactarates de formule I, tels que R1 = R2 = butyl, hexyl, octyl, décyl et R3 est hydroxy.

On porte à 85°C pendant 24 heures, plus une heure à 85°C (en distillant partiellement l'éthanol formé), 10 g de galactarate de diéthyle, 100 ml de DME, 1 ml d'acide sulfurique concentré et l'alcool désiré.

Le tableau II ci-après regroupe les quantités d'alcool mis en réaction et les rendements obtenus et le tableau III, les caractéristiques $RMN^{13}C$ des galactarates formés.

| Exemple n° | Composé | Quantité d'alcool mise en réaction | Rendement (%) |
|---|---|---|---|
| 11 | dibutyl | 6,9 ml butanol | 99,7 |
| 12 | dihexyl | 9,4 ml hexanol | 99,9 |
| 13 | dioctyl | 11,9 ml octanol | 99,4 |
| 14 | didécyl | 14,4 ml décanol | 99,7 |

TABLEAU II : Préparation d'alcoyl galactarates de formule I tel que R1 = R2 selon l'invention.

| Galactarate / Cn | Dibutyl* | Dihexyl* | Dioctyl* | Didécyl** |
|---|---|---|---|---|
| 1,6 | 173,54 | 173,51 | 173,52 | 173,99 |
| 2,5 | 71,23 | 71,23 | 71,22 | 71,87 |
| 3,4 | 70,08 | 70,11 | 70,11 | 70,98 |
| 1' | 63,66 | 63,96 | 63,97 | 63,84 |
| 2' | 30,16 | 30,77 | 31,11 | 30,95 |
| 3' | 18,45 | 28,06 | 28,11 | 28,67 |
| n-2' | | 24,86 | 25,20 | 24,92 |
| n-1' | | 21,86 | 21,95 | 21,76 |
| n | 13,42 | 13,68 | 13,79 | 13,10 |

* DMSO d6
** Pyridine d5

TABLEAU III :
Caractéristiques RMN13C
des composés décrits dans le tableau II.

Exemple n° 15 : Préparation du didodecyl galactarate (composé I tel que R1 = R2 = dodecYl et R3 est hydroxy).

On chauffe à 120°C pendant deux heures un mélange contenant 122 g de galactarate de diethyle, 171 g de dodecyl alcool, 2,5 g d'acide paratoluène sulfonique. Après distillation de l'éthanol formé pendant deux heures, on laisse le milieu réactionnel refroidir et on broye le produit. On obtient quantitativement 250 g de di Dodecyl galactarate.

RMN$^{13}$C : δ dans py-d5.

| Cn | 1,6 | 2,5 | 3,4 | 1′ | 2′ | 9′ | 10′ | 11′ | 12′ |
|---|---|---|---|---|---|---|---|---|---|
| ∂ | 174,59 | 72,88 | 71,94 | 65,04 | 31,96 | 20,90 | 25,98 | 22,699 | 13,95 |

Exemple n° 16 : Préparation du di Hexadecyl galactarate (composé I tel que R1 = R2 = Hexadecyl et R3 est hydroxy).

On chauffe à 120°C pendant 4 heures un mélange contenant 122 g de galactarate de dibutyl et 183,93 g d'Hexadecyl alcool, 2,5 g d'acide paratoluène sulfonique. Après distillation du butanol formé pendant 4 heures, on laisse refroidir et on broye le produit . On obtient quantitativement 250 g de Di Hexadecyl galactarate.

RMN$^{13}$C : δ dans py-d5.

| Cn | 1,6 | 2,5 | 3,4 | 1′ | 2′ | 13′ | 14′ | 15′ | 16′ |
|---|---|---|---|---|---|---|---|---|---|
| ∂ | 174,55 | 72,87 | 71,94 | 65,015 | 31,94 | 28,88 | 25,96 | 22,66 | 13,90 |

Exemple n° 17 : Préparation du galactarate d'éthyle et d'octyle (composé I tel que R1 = éthyl, R2 = octyl et R3 est hydroxy).

On chauffe à 85°C pendant 12 heures, un mélange contenant 10 g de galactarate de diéthyle, 4,9 g d'octanol, 100 ml de DME et 1 ml d'acide sulfurique concentré. Après distillation partielle de l'éthanol formé pendant 3 heures, on laisse encore le milieu réactionnel à reflux pendant 12 heures. On laisse encore le milieu réactionnel à reflux pendant 12 heures. On neutralise le milieu réactionnel par $NaHCO_3$, on filtre le sel obtenu pour récupérer quantitativement l'éthyl octyl galactarate.

Exemple n° 18 : Préparation du galactarate d'octyle et de décyle (composés I tel que R1 octyl, R2 = décyl et R3 est hydroxy).

On agite à 85°C pendant 24 heures, et en distillant l'éthanol formé pendant les 4 dernières heures, un mélange contenant 10 g (0,0375 mole) de galactarate de diéthyle, 4,9 g (0,0375 mole) d'octanol, 5,95 g (0,0375 mole) de décanol, 1 ml d'acide sulfurique concentré et 100 ml de diméthoxyéthane.

Après refroidissement, neutralisation par l'hydrogénocarbonate de sodium, filtration du sel formé, évaporation du filtrat, on obtient presque quantitativement le produit attendu (17,19 g; 98,4 % de rendement) sous forme d'un précipité blanc.

Exemple n° 19 : Préparation du butyl galactarate-1,4-lactone (composé I tel que R2 et R3 forment une liaison de valence entre les atomes qui les portent et R1 est butyle).

On chauffe à 85°C pendant 24 heures et en distillant totalement l'éthanol formé au cours de la réaction, un mélange contenant 10 g de galactarate de diéthyle, 3,85 g d'hexanol, 100 ml de DME et 1 g de $K_2CO_3$. Après filtration du catalyseur, on récupère quantitativement le butyl galactarate 1,4-lactone sous forme d'une huile.

RMN$^{13}$C : ∂ dans DMSO d6:

| Cn | 1 | 2 | 3 | 4 | 5 | 6 | 1′ | 2′ | 3′ | 4′ |
|---|---|---|---|---|---|---|---|---|---|---|
| ∂ | 171,29 | 72,09 | 67,18 | 80,30 | 73,41 | 173,90 | 64,30 | 30,03 | 18,39 | 13,37 |

Exemple n° 20 : Préparation du Octyl galactarate-1,4-lactone (composé I tel que R2 et R3 forment une liaison de valence entre les atomes qui les portent et R1 est octyle).

On chauffe à 120°C pendant 4 H en distillant l'éthanol formé au cours de la réaction, un mélange contenant 100 g de galactarate de diéthyl et 99 g d'octanol. Après refroidissement, on obtient quantitativement l'octyl galactarate-1,4-lactone.

RMN$^{13}$C : δ dans DMSO-$d_6$.

| Cn | 1 | 2 | 3 | 4 | 5 | 6 | 1′ | 2′ | 6′ | 7′ | 8′ |
|---|---|---|---|---|---|---|---|---|---|---|---|
| ∂ | 171,82 | 72,11 | 67,26 | 80,38 | 73,49 | 173,73 | 64,52 | 32,41 | 25,20 | 21,97 | 13,90 |

Exemple n°21 : Préparation du dodecyl galactarate-1,4-lactone (composé I tel que R2 et R3 forment une liaison de valence entre les atomes qui les portent et R1 est dodecyl).

On chauffe à 120°C pendant 6 H en distillant le butanol formé au cours de la réaction, un mélange contenant 100 g de galactarate de dibutyl et 116 g de dodecanol. Après refroidissement et broyage, on obtient quantitativement le produit.

RMN$^{13}$C : δ dans py-d5

| Cn | 1 | 2 | 3 | 4 | 5 | 6 | 1′ | 2′ | 10′ | 11′ | 12′ |
|---|---|---|---|---|---|---|---|---|---|---|---|
| ∂ | 172,37 | 73,90 | 69,35 | 82,088 | 75,464 | 175,15 | 65,49 | 33,65 | 16,12 | 22,82 | 14,16 |

Exemple n° 22 : Préparation de la 6-octadecyl galactarate-1,4-lactone (composé I tel que R2 et R3 forment une liaison de valence entre les atomes qui les portent, et R1 est octadecyle).

A 10 g de galactarate de dioctadecyl préparé selon l'invention, on ajoute 100 ml de DME et 1 g de $K_2CO_3$. Après 16 heures de chauffage à 85°C, on filtre le catalyseur et on concentre sous vide. Le produit brut obtenu est purifié sur colonne de silice (230-4 400 Mesh ASTM) en utilisant un gradient acétate d'éthyle / chloroforme-30/70-acétate d'éthyle pur. La 6-octadecyl galactarate 1,4-lactone est ainsi obtenue, après concentration, sous forme d'une poudre blanche.
Rendement 80%

RMN$^{13}$C : pyridine d5

| Cn | 1 | 2 | 3 | 4 | 5 | 6 | 1′ | 2′ | 3′ | 4′ | 17′ | 18′ |
|---|---|---|---|---|---|---|---|---|---|---|---|---|
| ∂ | 173,30 | 74,00 | 69,43 | 82,20 | 75,57 | 175,38 | 65,59 | 33,78 | 32,11 | 29,97 | 22,93 | 14,27 |

Exemple n° 23 : Préparation de l'octyl galactarate-1,4 lactone (composé I tel que R2 et R3 forment une liaison de valence entre les atomes qui les portent, et R1 octyle).

Un mélange de 10 g de la monolactone de l'acide mucique, 4,6 g de n. octanol, 1 g de carbonate de potassium et 100 ml de DME est porté à 85°C pendant 12 heures. Après filtration du catalyseur et concentration du filtrat, on récupère avec 90% du rendement la galactarate lactone attendue.

Exemple n° 24 : Préparation de l'hexyl galactarate de potassium (composé I tel que R2 et R3 forment une liaison de valence entre les atomes qui les portent et R1 est hexyle).

Un mélange de 10 g d'hexyl galactarate-1,4 lactone préparé selon l'invention, de 1 ml d'eau, de 100 ml de DME et de 1,6 g de $k_2CO_3$ sont portés à 85°C pendant 12 heures après filtration du catalyseur et concentration sous vide, on récupère avec 98 % de rendement, l'hexyl galactarate 1,4-lactone attendue.

RMN$^{13}$C : DMSO d6

| Cn | 1 | 2 | 3 | 4 | 5 | 6 | 1′ | 2′ | 3′ | 4′ | 5′ | 6′ |
|---|---|---|---|---|---|---|---|---|---|---|---|---|
| ∂ | 174,65 | 73,55 | 69,21 | 81,17 | 73,85 | 172,67 | 60,58 | 32,37 | 31,08 | 25,05 | 22,02 | 13,79 |

Exemple n° 25:

On prépare un composition lessivielle en mélangeant dans de l'eau, les pourcentages étant exprimés en poids.

- 3 % de Neodol 45-11 (Shell) qui est un agent tensio-actifs non ionique constitué d'un produit de condensation de l'oxyde d'éthylène comprenant 1 mole d'alcool gras supérieur ayant de 14 à 15 atomes de carbone et 11 moles d'oxyde d'éthylène (SHELL CHEMICAL COMPANY),
- 3% de galactarate de didecyl de l'exemple 14, 1,5% de Rewoteric AMDML (REWO) qui est un agent tensio-actif amphotère constitué d'une bétaïne de formule

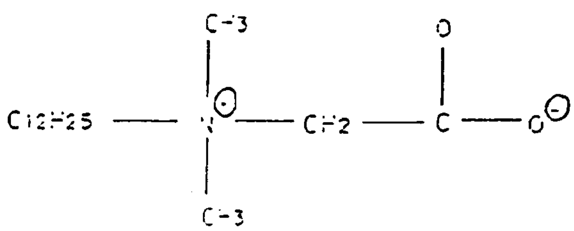

- 3 % de silicate de sodium,
- 42 % de tripolyphosphate de sodium,
- 0,3 % de brillanteur optique constitué par le produit Tinopal ATS-X (Ciba-Geigy),
- 20 % de perborate de sodium,
- 4,1 % de tétraacétyléthylènediamine servant d'activateur de blanchiment,
- 5,5 % de chlorure de distéaryldiméthylammonium servant d'agents adoucissant,
- 3,0 % de méthylsulfate de N-méthyl-N-(2-hydroxyéthyl)-N-suifalkyl-N'-méthyl-N'-bis(2-hydroxyéthyl)propylènediammonium servant d'adjuvant de traitement,
- 1,0 % d'alcalase 2T,
- 2,0 % de carboxyméthylcellulose sodique,
- 0,5 % de parfum et,
- 0,5 % d'acide éthylènediaminetétraacétique.

On lave des vêtements en coton à l'aide de cette composition. On constate une bonne blancheur avec de bonnes propriétés adoucissantes.

Exemple n° 26:

On prépare un shampooing en mélangeant :

1) 4,5 g du 6 octadecyl galactarate-1,4-lactone de l'exemple 18, tensio-actif non ionique émulsionnant.
2) 50 g de alkylétheroxydesulfate de sodium et de magnésium, tensio-actif anionique détergent.
3) 2 g d'alkylamidodiméthylbétaïne, tensio-actif amphotère démêlant.
4) 3 g de laurate de sorbitant éthoxylé, tensio-actif non ionique émulsionnant.
5) 1 g de chlorure de sodium, épaississant.
6) 0,1 g de conservateur.
7) 0,2 g de parfum.
8) Eau déminéralisée pour faire 100 g.

**Revendications**

**1- ALCOYL GALACTARATES DE FORMULE :**

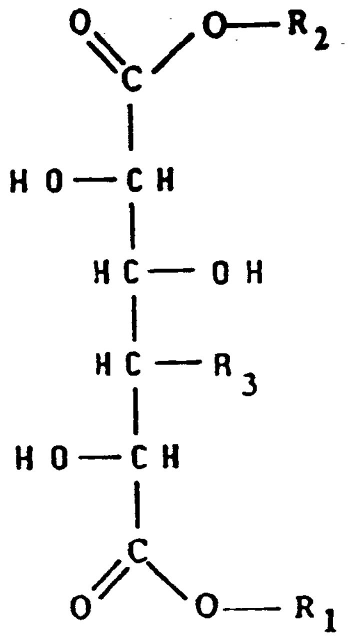

(I)

dans laquelle R1 est alcoyle ou alcényle ayant de 1 à 22 atomes de carbone et, ou bien R2 est, quand il est différent de R1, l'hydrogène, alcoyle ou alcényle ayant de 1 à 22 atomes de carbone, et quand il est identique à R2, alcoyle ou alcényle ayant de 7 à 22 atomes de carbone, et R3 est hydroxy, ou bien R3 forme avec R2 une liaison de valence entre les atomes qui les portent, et leurs sels avec des métaux alcalins, alcalino-terreux ou d'ammonium.

2- PROCEDE DE PREPARATION D'ALCOYL GALACTARATES SUIVANT LA REVENDICATION 1 CARACTERISE EN CE QU'IL CONSISTE :

+ Pour préparer les alcoyl galactarates de formule (I) dans lesquels R1 et R2 sont identiques, à transestérifier un galactarate de dialcoyle symétrique ayant une condensation en carbone de chacune de ses chaînes alcoyles égale à n, n entier inférieur à 7 et de préférence égale à 2.

+ Pour préparer les alcoyl galactarates de formule (I) dans lesquels R1 n'est pas l'hydrogène et est différent de R2, à transestérifier un galactarate de dialcoyle symétrique ayant une condensation en carbone de chacune de ses chaînes égales à n, n entier inférieur à 7, et de préférence égale à 2, ou bien par au plus un équivalent molaire d'un alcool de formule $R_2OH$, $R_2$ ayant une condensation en carbone supérieure à n, en présence d'un catalyseur acide ; ou bien par un mélange d'au moins deux alcools de formules respectives $R_1OH$ et $R_2OH$, tant R1 que R2 ayant une condensation en carbone supérieure à n, et la somme des moles des alcools de formules $R_1OH$ et $R_2OH$ engagés dans la réaction étant supérieure au nombre de moles de dialcoyl galactarate symétrique mis en réaction et de préférence égal au double, ceci en présence d'un catalyseur acide.

+ Pour préparer les alcoyl galactarates cyclisés de formule I dans lesquels R3 forme avec R2 une liaison de valence,

. ou bien à transestérifier un galactarate de dialcoyle symétrique ayant une condensation en carbone de chacune de ses chaînes égale à n, n entier inférieur à 7, et de préférence égale à 2, par au plus un équivalent molaire d'un alcool R1OH, R1 ayant une condensation en carbone supérieure à n, en présence d'un catalyseur basique, puis à éliminer totalement par distillation, l'alcool de formule $C_nH_{2n+1}OH$ résultant de la transestérification et de la cyclisation,

. ou bien, à saponifier unilatéralement puis à cycliser, en milieu basique, un galactarate de dialcoyle symétrique ayant une condensation en carbone de chacune de ses chaînes égale à m, m entier compris entre 2 et 22 et obtenu selon l'invention,

. ou bien, à estérifier en milieu basique par un équivalent molaire d'un alcool de formule R1OH, R1 ayant une condensation en carbone allant de 1 à 22 atomes, la monolactone de l'acide galactarique.

. ou bien à chauffer un dialcoyle galactarate symétrique de formule I dans un solvant organique.

+ Pour préparer les alcoyl galactarate de formule (I) dans lesquels R1 est l'hydrogène, soit à mettre en

présence d'eau un alcoyle galactarate cyclisé dans lequel R3 forme avec R2 une liaison de valence, soit à traiter ce dernier par un équivalent d'une base en solution aqueuse pour obtenir la forme sel de l'acide correspondant à l'ouverture de la fonction lactone et, pour préparer les sels, à mettre les alcoyl galactarates de formule I en contact avec le carbonate, l'hydrogénocarbonate ou l'hydroxyde correspondant.

**3** - L'UTILISATION DES ALCOYL GALACTARATES TELS QUE DEFINIS A LA REVENDICATION 1 COMME AGENTS TENSIO-ACTIFS.

**4** - L'UTILISATION DES ALCOYL GALACTARATES TELS QUE DEFINIS A LA REVENDICATION 1 DANS DES APPLICATIONS LESSIVIELLES ET COSMETIQUES.

**5** - L'UTILISATION DES ALCOYL GALACTARATES TELS QUE DEFINIS A LA REVENDICATION 1 DANS LA PREPARATION DE LIPOSOMES.

**Revendications pour l'Etat contractant suivant: ES**

**1.** PROCEDE DE PREPARATION D'ALCOYL GALACTARATES DE FORMULE:

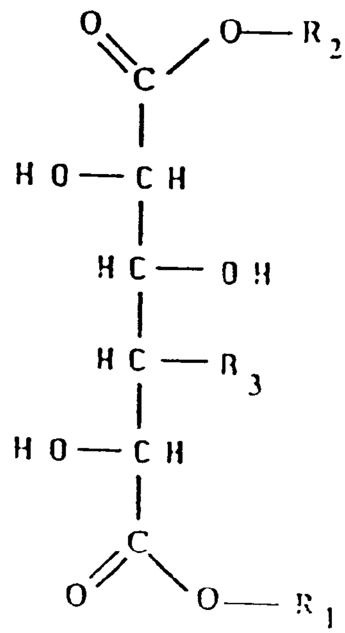

(I)

dans laquelle R1 est alcoyle ou alcényle ayant de 1 à 22 atomes de carbone et, ou bien R2 est, quand il est différent de R1, l'hydrogène, alcoyle ou alcényle ayant de 1 à 22 atomes de carbone, et quand il est identique à R2, alcoyle ou alcényle ayant de 7 à 22 atomes de carbone, et R1 est hydroxy, ou bien R1 forme avec R2 une liaison de valence entre les atomes qui les portent, et leurs sels avec des métaux alcalins, alcalino-terreux ou d'ammonium, caractérisé en ce qu'il consiste:

- Pour préparer les alcoyl galactarates de formule (1) dans lesquels R1 et R2 sont identiques, à transestérifier un galactarate de dialcoyle symétrique ayant une condensation en carbone de chacune de ses chaînes alcoyles égale à n, n entier inférieure à 7 et de préférence égale à 2.
- Pour préparer les alcoyl galactarates de formule (I) dans lesquels R1 n'est pas l'hydrogène et est différent de R2, à transestérifier un galactarate de dialcoyle symétrique ayant une condensation en carbone de chacune de ses chaînes égales à n, n entier inférieur à 7, et de préférence égale à 2, ou bien par au plus un équivalent molaire d'un alcool de formule $R_2OH$, $R_2$ ayant une condensation en carbone supérieure à n, en présence d'un catalyseur acide ; ou bien par un mélange d'au moins deux alcools de formules respectives $R_1OH$ et $R_2OH$, tant R1 que R2 ayant une condensation en carbone supérieure à n, et la somme des moles des alcools de formules $R_1OH$ et $R_2OH$ engagés dans la réaction étant supérieure au nombre de moles de dialcoyl galactarate symétrique mis en réaction et de préférence égal au double, ceci en présence d'un catalyseur acide.
- Pour préparer les alcoyl galactarates cyclisés de formule I dans lesquels R3 forme avec R2 une liaison

de valence,

. ou bien à transestérifier un galactarate de dialcoyle symétrique ayant une condensation en carbone de chacune de ses chaînes égale à n, n entier inférieur à 7, et de préférence égale à 2, par au plus un équivalent molaire d'un alcool R1OH, R1 ayant une condensation en carbone supérieure à n, en présence d'un catalyseur basique, puis à éliminer totalement par distillation, l'alcool de formule $C_nH_{2n+1}OH$ résultant de la transestérification et de la cyclisation,

. ou bien, à saponifier unilatéralement puis à cycliser, en milieu basique, un galactarate de dialcoyle symétrique ayant une condensation en carbone de chacune de ses chaînes égale à m, m entier compris entre 2 et 22 et obtenu selon l'invention,

. ou bien, à estérifier en milieu basique par un équivalent molaire d'un alcool de formule R1OH, R1 ayant une condensation en carbone allant de 1 à 22 atomes, la monolactone de l'acide galactarique.

. ou bien à chauffer un dialcoyle galactarate symétrique de formule I dans un solvant organique.

- Pour préparer les alcoyl galactarate de formule (I) dans lesquels R1 est l'hydrogène, soit à mettre en présence d'eau un alcoyle galactarate cyclisé dans lequel R3 forme avec R2 une liaison de valence, soit à traiter ce dernier par un équivalent d'une base en solution aqueuse pour obtenir la forme sel de l'acide correspondant à l'ouverture de la fonction lactone et, pour préparer les sels, à mettre les alcoyl galactarates de formule I en contact avec le carbonate, l'hydrogénocarbonate ou l'hydroxyde correspondant.

**2.** L'utilisation des alcoyl galactarates tels que définis à la revendication 1 comme agents tensio-actifs.

**3.** L'utilisation des alcoyl galactarates tels que définis à la revendication 1 dans des applications lessivielles et cosmétiques.

**4.** L'utilisation des alcoyl galactarates tels que définis à la revendication 1 dans la préparation de liposomes.

Office européen des brevets

# RAPPORT DE RECHERCHE EUROPEENNE

Numero de la demande
EP 92 40 2075

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| X,D | DE-C-878 863 (FARBWERKE HOECHST)<br>* page 1, ligne 25 *<br>--- | 1 | C07C69/675<br>C07C67/03<br>C11D1/04<br>C11D1/66<br>A61K7/075<br>C07C67/30 |
| A | JOURNAL OF THE AMERICAN CHEMICAL SOCIETY.<br>vol. 70, no. 7, Juillet 1948, GASTON, PA US<br>page 2609 'Esters of mucic acid'<br>* whole document *<br>----- | 1 | |

| DOMAINES TECHNIQUES RECHERCHES (Int. Cl.5 ) |
|---|
| C07C |

Le présent rapport a été établi pour toutes les revendications

| Lieu de la recherche | Date d'achèvement de la recherche | Examinateur |
|---|---|---|
| LA HAYE | 05 OCTOBRE 1992 | KINZINGER J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)